# EUROPÄISCHE PATENTSCHRIFT

(11) **EP 1 562 613 B1**
(45) Veröffentlichungstag und Bekanntmachung des Hinweises auf die Patenterteilung: **12.03.2008**
(21) Anmeldenummer: 03779534.1
(22) Anmeldetag: 19.11.2003
(51) Int. Cl.: A61K 31/70, A61K 31/455, A61K 31/198, A61K 31/355, A61K 31/385, A61P 39/06

(54) **ZUSAMMENSETZUNG UMFASSEND NADH/NADPH**
NADH/NADPH-CONTAINING COMPOUND
COMPOSITION COMPRENANT NADH/NADPH

(30) Priorität: 19.11.2002 AT 17342002
(43) Veröffentlichungstag der Anmeldung: 17.08.2005
(73) Patentinhaber: NUTROPIA Ernährungsmedizinische Forschungs GmbH, 5580 Unternberg (AT)
(72) Erfinder: SADEGHI, Behzad, A-1010 Wien (AT); KÖSSLER, Peter, A-5571 Mariapfarr (AT); FUCHS, Norbert, A-5571 Mariapfarr (AT)
(74) Vertreter: Sonn & Partner Patentanwälte
(86) Internationale Anmeldenummer: PCT/AT2003/000346
(87) Internationale Veröffentlichungsnummer: WO 2004/045626

(56) Entgegenhaltungen:
- RU-C- 2 143 212
- PATENT ABSTRACTS OF JAPAN vol. 2000, no. 01, 31. Januar 2000 (2000-01-31) & JP 11 292737 A (SHISEIDO CO LTD), 26. Oktober 1999 (1999-10-26)
- PATENT ABSTRACTS OF JAPAN vol. 009, no. 287 (C-314), 14. November 1985 (1985-11-14) & JP 60 132987 A (RIKEN VITAMIN OIL KK), 16. Juli 1985 (1985-07-16)
- PATENT ABSTRACTS OF JAPAN vol. 009, no. 082 (C-275), 11. April 1985 (1985-04-11) & JP 59 216824 A (YOSHIROU SHIMURA), 6. Dezember 1984 (1984-12-06)

## Beschreibung

Die vorliegende Erfindung betrifft eine Zusammensetzung sowie ein Verfahren zu deren Herstellung und die Verwendung der Zusammensetzung.

Der Mensch benötigt als heterotrophes Wesen täglich Makromoleküle (Eiweiß, Fette Kohlenhydrate), um seine energetischen und stofflichen Bedürfnisse zu decken. Im Rahmen des Energiestoffwechsels verbrennt die humane Zelle die Spaltprodukte der Makronährstoffe, indem sie Elektronen dieser Spaltprodukte auf den molekularen Atmungs-Sauerstoff überträgt. Dabei wird Wärmeenergie und biochemische Speicherenergie in Form von ATP und anderen energiereichen Phosphaten als Depot gebildet. Ein Großteil dieser Spaltprodukte wird aber auch zur Biosynthese und Regeneration neuer biologischer Strukturen (z.B. von Enzymen, Hormonen, Körperzellen, Bindegewebssubstanz) herangezogen. Auch diese Aufbau-Vorgänge benötigen sowohl Elektronen als auch biochemische Energie (z.B. ATP), die aus Verbrennungsvorgängen frei wurde. Sowohl die Oxidation (bei der Energie durch die Zerstörung von chemischen Strukturen frei wird) als auch die Reduktion (bei der neue biologische Strukturen durch Energieaufwand aufgebaut werden) sind Stoffwechselvorgänge, bei denen Elektronen aus den Makronährstoff-Molekülen übertragen werden. Somit sind sämtliche Stoffwechsel-Vorgänge im menschlichen Körper Oxidations- und Reduktions-Vorgänge, sogenannte Redox-Vorgänge, wobei hierfür "gesundheitsfördernde" Antioxidantien wichtig sind.

Nachdem bei biochemischen Vorgängen Elektronen nicht isoliert übertragen werden können, benötigt die Zelle Überträgermoleküle, die man auch als Elektronen-Carrier bezeichnet. Prinzipiell ist jedes Atom, jedes Molekül, das Elektronen an einen Reaktionspartner überträgt, ein Elektronen-Carrier bzw. - in Relation zu jenem Partner, auf den das Elektron übertragen wird - ein Elektronen-Donator bzw. ein Reduktionsmittel bzw. ein Antioxidans. Das Potential, Elektronen zu übertragen, ist von Molekül zu Molekül verschieden. Dieses Standard-Redoxpotential ist umso niedriger, je stärker der Elektronendruck von diesem Molekül ausgeübt wird und um so höher, je geringer dieser Elektronendruck ist. Moleküle mit niedrigem Standard-Redoxpotential tragen energiereiche Elektronen, die entweder in biochemische Energie oder in den Aufbau neuer biologischer Strukturen mit hoher Ordnung einfließen, d.h., dass die Vitalität des menschlichen Körpers letztlich davon abhängt, wie hoch der Anteil an energiereichen Elektronen in der Nahrung ist. NADH zählt beispielsweise aufgrund seines äußerst niedrigen Standard-Redoxpotentials von -320mV zu den wichtigsten und energiereichsten Elektronen-Carriern des menschlichen Stoffwechsels. NADH überträgt die Elektronen (als gebundenes "Hydrid") vorwiegend zum Zwecke der Energiegewinnung (unter gleichzeitiger Bildung von ATP) auf molekularen Atmungssauerstoff. Dieser - als oxidative Phosphorylierung oder Atmungskette bezeichnete - Reaktionsablauf findet in den Mitochondrien der Zellen statt. NADH ist somit gleichsam das Symbol für zelluläre Energie.

NADH fördert kognitive, intellektuelle und motorische Leistungen bei Morbus Parkinson und Morbus Alzheimer, ebenso wie bei Müdigkeit, Antriebslosigkeit und beim Chronischen Müdigkeitssyndrom. NADH verbessert auch die Lebensfreude, die Leistungsbereitschaft, vereinzelt wird auch berichtet, dass Libido- und Potenzstörungen durch NADH beseitigt werden können. Depressionen, Lern- und Konzentrationsschwäche können ebenso Folge einer eingeschränkten endogenen NADH-Synthese sein wie eine reduzierte körperliche Leis tungsfähigkeit unter körperlicher Belastung und im Leistungssport. Nicht zuletzt fördert NADH die Synthese- und Entgiftungsleistungen der Leber.

Es gibt jedoch eine Reihe weiterer solcher "gesundheitsfördernder" Antioxidantien, die großteils im Organismus selbst produziert werden. Prinzipiell ist also der menschliche Organismus in der Lage diese Antioxidantien, wie z.B. NADH, selbst aufzubauen. NADH entsteht über mehrere Stoffwechselschritte enzymatisch und unter hohem Energieaufwand aus Niacin bzw. aus dessen Vorstufe L-Tryptophan und aus dem phosphorhaltigen Zucker 5-Phosphoribosyl-1-Pyrophosphat(PRPP), einer Ribose mit drei Phosphatresten. Auf das daraus gebildete Nikotinat-Ribonucleotid wird ein Molekülrest aus ATP (nämlich AMP) übertragen. Als letzter Syntheseschritt wird eine Aminogruppe des Glutamins transferiert, woraus NAD⁺ hervorgeht. Wird auf diese Moleküle ein weiteres Phosphat (aus ATP) angedockt, so entsteht daraus (das "Regenerationsmolekül") NADP. Die eigentliche biologische Aktivierung von NAD⁺ - durch Übertragung eines Hydrids aus Nährstoffmolekülen - erfolgt ebenfalls durch ATP-Abbau, also unter hohem Energieverbrauch. Niacin und Glutamin fördern somit die endogene NADH-Synthese, vorausgesetzt, der zelluläre Pool an ATP und energiereichen Syntheseenzymen ist ausreichend gefüllt.

Prinzipiell wäre es aber auch möglich, die gewünschten und für die Gesundheit eines Orgaismus wichtigen Antioxidantien auch exogen über die Nahrung zuzuführen. Diese Antioxidantien sind aufgrund ihres sehr geringen Standard-Redoxpotentials allerdings äußerst empfindlich gegenüber Sauerstoff und daher in reduzierter Form praktisch nicht verfügbar.

Die Aufgabe der vorliegenden Erfindung liegt daher darin, gesundheitsfördernde Antioxidantien mit einem geringen Redoxpotential von unter -180mV in einer lagerstabilen Form zur Verfügung zu stellen, sodass die Antioxidantien zusätzlich zur endogenen Produktion auch exogen aufgenommen werden können. Dabei soll die Lagerstabilität so hoch sein, dass die Antioxidantien über einen Zeitraum von zumindest 6 Monaten vor einem oxidativen und chemischen Zerfall geschützt sind.

Diese Aufgabe wird erfindungsgemäß durch eine Zusammensetzung gelöst, die dadurch gekennzeichnet ist, dass sie zumindest ein isoliertes, gesundheitsförderndes Antioxidans A mit einem Redoxpotential von unter -180mV, ausgewählt aus der Gruppe bestehend aus NADH, NADPH, FADH₂, FADH und FMNH, und zumindest ein isoliertes, das Antioxidans A stabilisierende Antioxidans B mit einem Standard-Redoxpotential, das unter dem Standard-Redoxpotentials des Antioxidans A liegt, umfasst, wobei das Antioxidans B ein Chlorophyll und/oder ein reduziertes Ferredoxin ist.

Durch das Vorsehen eines Antioxidans B mit einem geringeren Standard-Redoxpotential als das des Antioxidans A wurde überraschenderweise festgestellt, dass das Antioxidans A ausreichend lagerstabil bleibt und vor einem Zerfall geschützt ist, im Gegensatz zu herkömmlichen Zusammensetzungen:

Beispielsweise ist in der EP 1 161 884 A1 offenbart, dass ein Nahrungsmittelzusatz, umfassend NADH, durch Vitamin E stabilisiert wird. Jedoch weist Vitamin E ein höheres Standard-Redoxpotential als das erfindungsgemäße Antioxidans B auf, beispielsweise weist Tocopherol ein Standard-Redoxpotential von +300mV auf.

Die US 5,332,727 und die US 5,952,312 betreffen eine Zusammensetzung umfassend NADH bzw. NADPH und als Stabilisator NaHCO₃, Ascorbinsäure, Natriumascorbat, Tocopherol, Tocopherolacetat oder Polyvinylpyrolidon. Auch diese Stabilisatoren weisen jedoch ein Standard-Redoxpotential auf, das höher ist als das des erfindungsgemäßen Antioxidans B, und sie sind nicht geeignet, um beispielsweise NADH bzw. NADPH über ein erniedrigtes Standard-Redoxpotential optimal zu stabilisieren. Ascorbinsäure etwa weist ein Redoxpotential von +80mV auf.

Die in der Zusammensetzung vorgesehenen Antioxidantien können dabei sowohl synthetischen als auch natürlichen Ursprungs sein, wichtig dabei ist, dass die Antioxidantien isoliert, d.h. gereinigt, vorgesehen sind, wobei wie bereits angeführt ein einzelnes isoliertes Antioxidans oder ein Gemisch von isolierten Antioxidantien vorgesehen sein kann, wobei zumindest eines der Antioxidantien B ein Standard-Redoxpotential aufweist, das niedriger als jenes des zu schützenden Antioxidans A ist. Dabei wird unter "isoliert" weiters verstanden, dass das gereinigte Antioxidans in der Zusammensetzung in einer Konzentration vorhanden ist, die höher als in natürlichen, d.h. in der Natur vorkommenden, Gemischen. Solche Konzentrationen sind vorzugsweise über 20%, bevorzugterweise über 40%, noch bevorzugter über 60% der Zusammensetzung ausgenommen Stabilisatoren, Salze und weitere Hilfsstoffe. Das Redoxpotential des Antioxidans B kann beispielsweise unter -190mV, unter -320mV bzw. unter -600mV sein.

Unter dem Begriff "Antioxidans A" sind solche mit einem Redoxpotential von unter -180mV zu verstehen, die gesundheitsfördernd sind, wobei der Begriff "gesundheitsfördernd" bedeutet, dass das Antioxidans für ein Funktionieren eines menschlichen oder tierischen Organismus wichtig ist. Dabei sind vorzugsweise solche Antioxidantien darunter zu verstehen, die bereits im menschlichen oder tierischen Organismus hergestellt werden, die jedoch aufgrund von angeborenen Erkrankungen oder aufgrund von äußeren Einflüssen in zu geringen Konzentrationen im Körper selbst endogen hergestellt werden, so dass eine exogene Zufuhr für die optimale Gesundheit wichtig ist. Solche Antioxidantien A sind NADH, NADPH, FADH₂, FMNH₂, FADH und FMNH.

Unter den Begriff "Antioxidans B" ist im Rahmen der vorliegenden Anmeldung ein Antioxidans zu verstehen das ein geringeres Standard-Redoxpotential aufweist, als das in der Zusammensetzung vorhandene Antioxidans A, so dass das Antioxidans A durch das Antioxidans B stabilisiert wird. Dabei wird das Antioxidans in erster Linie keinen direkten Einfluss auf die Gesundheit des menschlichen oder tierischen Organismus ausüben.

Ist lediglich ein Antioxidans A in der Zusammensetzung vorhanden, etwa FADH, mit einem Standard-Redoxpotential von -190mV, so reicht ein Antioxidans B, mit einem Standard-Redoxpotential von unter -190mV in der Zusammensetzung aus, um FADH über das Redoxpotential zu stabilisieren. Ist andererseits neben dem FADH weiters ein zweites oder sind mehrere weitere Antioxidantien A in der Zusammensetzung vorhanden, die ein niedrigeres Redoxpotential aufweisen, wie etwa FADH₂ (-220mV) und/oder FMNH₂ (-220mV), so muss zumindest ein Antioxidans B mit einem Standard-Redoxpotential von unter -220mV in der Zusammensetzung vorhanden sein. Selbsverständlich kann auch zusätzlich ein Antioxidans B mit einem Redoxpotential von unter -190mV vorhanden sein. Ob ein oder mehrere Antioxidantien B in der Zusammensetzung vorhanden sind, wird von Fall zu Fall zu entscheiden sein, die von den Anwendungsmöglichkeiten aufgrund des Herstellungsprozesses, Kostengründen und anderen Faktoren abhängen. Wichtig ist jedenfalls, dass zur Stabilisierung des Antioxidans A mit dem geringsten Redoxpotential immer ein Antioxidans B mit einem noch geringeren Redoxpotential vorhanden ist.

Der Unterschied zwischen dem Antioxidans A und dem Antioxidans B liegt darin, dass das Antioxidans A einen gesundheitsfördernden Effekt aufweist, das Antioxidans B jedoch nicht notwendigerweise sondern in erster Linie dazu dient, das Antioxidans A zu stabilisieren.

Dabei wird das Antioxidans A ausgewählt aus der Gruppe bestehend aus NADH, NADPH, FADH₂, FMNH₂, FADH bzw. FMNH. Diese Antioxidantien weisen folgende Redoxpotentiale auf:

FADH₂: -220mV, FMNH₂: -220mV, FADH: -190mV, FMNH: -190mV und NADH: -320mV. Diese Antioxidantien sind besonders wichtig für die Gesundheit des menschlichen bzw. tierischen Organismus und besitzen Standard-Redoxpotentiale, die geringer sind als -180mV. Diese Stoffe sind besonders empfindlich und es ist das aus diesem Grund problematisch diese Stoffe in einer lagerstabilen Form zur Verfügung zu stellen ohne, dass sich die Antioxidantien z.B. chemisch verändern und ohne gesundheitsschädliche Konservierungsmittel zusetzen zu müssen. Selbstverständlich können auch all diese Antioxidantien gemeinsam in der Zusammensetzung vorhanden sein. Dabei ist es lediglich wichtig, dass eine ausreichende Menge an Antioxidans B vorhanden ist, um alle Antioxidantien A zu stabilisieren.

Die Zusammensetzung kann dabei in jeglicher für ihre Verabreichung günstigen Form vorgesehen sein, beispielsweise in Form von üblichen Lebensmitteln, Nahrungsergänzungsmitteln, diätetisch Lebensmitteln, Arzneimitteln, etc.

Das Antioxidans B ist ein Chlorophyll und/oder ein reduziertes Ferredoxin. Es hat sich überraschenderweise herausgestellt, dass sich Chlorophyll besonders gut als Schutz gegen den Zerfall von Antioxidantien mit einem Redoxpotential von unter -180mV eignet, wobei eine einzige oder aber auch mehrere Chlorophyllarten vorgesehen werden können, z.B. Chlorophyll A, B, C und/oder D, wobei die Chlorophylle aus dem Photosystem I ein niedrigeres Redoxpotential aufweisen als die Chlorophylle aus dem Photosystem II.

Besonders bevorzugt umfasst die Zusammensetzung weiters eine sauerstoffmaskierende Substanz. Wird zusätzlich zum Antioxidans B weiters eine sauerstoffmaskierende Substanz in der Zusammensetzung vorgesehen, wird dadurch der Kontakt des Antioxidans A mit Sauerstoff, und dadurch ein Oxidationsprozess des Antioxidans A, verhindert. Auch hier ist es selbstverständlich möglich, nicht nur eine sauerstoffmaskierende Substanz sondern zwei oder mehrere verschiedene sauerstoffmaskierende Substanzen in der Zusammensetzung vorzusehen, wobei unter dem Begriff "sauerstoffmaskierende Substanz" jeglicher Stoff oder jegliche Stoffzusammensetzung zu verstehen ist, die den Kontakt zwischen Antioxidans A und Sauerstoff reduziert bzw. weitgehend verhindert.

Vorzugsweise ist die sauerstoffmaskierende Substanz eine ölhältige Substanz. Ölhältige Substanzen eignen sich als sauerstoffmaskierende Substanz der erfindungsgemäßen Zusammensetzung besonders gut, da sie effizient das Inkontaktkommen von Sauerstoff mit dem Antioxidans A der Zusammensetzung verhindern bzw. zumindest stark herabsetzen und weiters auch, da eine Reihe von ölhältigen Substanzen bekannt sind, die gesundheitsfördernd sind. Auch hier ist es wiederum selbstverständlich möglich, dass nicht nur eine ölhältige Substanz, sondern verschiedene ölhältige Substanzen, etwa verschiedene ungesättigte Fettsäuren enthaltende Öle bzw. Öle unterschiedlicher Herkunft verwendet werden können.

Dabei ist es besonders günstig, wenn die sauerstoffmaskierende Substanz eine ölhältige Substanz, umfassend zumindest ein weiteres Antioxidans C, ist. Dadurch wird der Schutz des Antioxidans A zusätzlich erhöht, da der oxidative Zerfall des Antioxidans A nicht nur durch das Antioxidans B verhindert wird, sondern auch in doppelter Weise durch die ölhältige Substanz, erstens, da der Kontakt zwischen dem Antioxidans A und Sauerstoff verhindert oder weitgehend reduziert wird und im Fall der Gegenwart von Sauerstoff zweitens, indem dieser bereits durch das Antioxidans C in der ölhältigen Substanz reduziert wird.

Besonders bevorzugt umfasst die sauerstoffmaskierende Substanz als Antioxidans C Vitamin E, insbesondere Tocotrienol. Vitamin E ist ein Antioxidans mit zusätzlichen gesundheitsfördernden Eigenschaften, etwa cholesterinsenkenden und zellschützenden Eigenschaften. Dabei kann eine Art Vitamin E vorgesehen werden, etwa eine Tocopherol-Art, als auch zumindest zwei oder mehr Vitamin E-Arten. Dabei ist es jedoch besonders günstig, wenn Tocotrienol vorgesehen wird, da Tocotrienol ein 50- bis 1000fach höheres antioxidatives Potential als synthetische Tocopherole aufweist. Tocotrienole spielen als lipophile Antioxidantien eine wichtige biologische Rolle im Rahmen des antioxidativen Schutzes von Zellkernen (genetisches Material), der Mitochondrien (zelluläre Energieversorgung), des endoplasmatischen Retikulums (zelluläre Syn theseleistung), sowie an der Zellmembran (Stabilität und Lebensdauer von Geweben). Der ernährungsmedizinische Anwendungsbereich von Tocotrienolen umfasst beispielsweise das Immunsystem, das kardiovaskuläre System, den Muskel/Sehnen/Gelenks-Apparat, die Leber als Entgiftungsorgan, die Haut sowie regenerative Prozesse des Nervensystems. Selbstverständlich können sowohl Tocotrienole als auch Tocopherole in der sauerstoffmaskierenden Substanz vorgesehen sein.

Eine besonders günstige Zusammensetzung wird dadurch zur Verfügung gestellt, dass das Antioxidans A und das Antioxidans B in einem Verhältnis von zwischen 10:1 und 1:10, vorzugsweise zwischen 3:1 und 1:3, vorgesehen sind.

Es hat sich herausgestellt, dass diese Verhältnisse optimal für den Schutz des Antioxidans A sind, wobei das Verhältnis von insbesonders 1:1 bis 1:3 einen umfassenden Schutz des Antioxidans A ergibt.

Vorzugsweise umfasst die Zusammensetzung weiters ein oder mehrere Hilfsmittel. Die Hilfsmittel können - je nach Verwendungsart der Zusammensetzung - pharmazeutisch akzeptable Träger, Emulgatoren, Stabilisatoren, Farbstoff, Geschmacksstoffe, weitere pharmazeutische Wirkstoffe, lebensmitteltechnische Hilfsstoffe und Ähnliches sein. Als Träger eignet sich beispielsweise Kieselsäure besonders gut, da diese neutral ist und keine Wirkung auf das Redoxpotential der Inhaltsstoffe ausübt.

Ein weiterer Aspekt der vorliegenden Erfindung betrifft die Verwendung der erfindungsgemäßen Zusammensetzung wie oben beschrieben zur Nahrungsergänzung. Auf diese Weise kann auch ohne Erkrankung und ohne ärztliche Behandlung, wie z.B. im Fall von Müdigkeit, wie sie bei vielen Personen, insbesondere in Form von Frühjahrsmüdigkeit auftritt, das Antioxidans A einfach in Form einer Nahrungsergänzung eingenommen werden. Aber auch bei erhöhten Leistungen, wie bei vermehrtem Sport, bei Prüfungsstress, etc. kann die Zusammensetzung in Form einer Nahrungsergänzung eingenommen werden. Hierbei wird die Zusammensetzung in üblichen Formen für Nahrungsergänzungsmittel, Lebensmitteln bzw. diätische Lebensmittel zur Verfügung gestellt.

Ein weiterer Aspekt der vorliegenden Erfindung betrifft die Verwendung der erfindungsgemäßen Zusammensetzung, wie oben definiert, als Arzneimittel. Da die Einnahme des Antioxidans A, insbesondere auch bei Erkrankungen, wie chronischer Müdigkeit, bei Depressionen oder motorischen Schwächen, einen positiven Einfluss ausübt, ist es günstig, die erfindungsgemäße Zusammensetzung in Form eines Arzneimittels vorzusehen, wobei auch hier übliche Arzneimittel-Formen vorgesehen sind.

Ein weiterer Aspekt der vorliegenden Erfindung betrifft die Verwendung einer Zusammensetzung gemäß der vorliegenden Erfindung wie oben beschrieben zur Herstellung eines Mittels ausgewählt aus der Gruppe bestehend aus einem Mittel zur Förderung der oxidativen Phosphorylierung, einem Mittel zur Förderung der kognitiven, intellektuellen bzw. motorischen Leistungen, einem Mittel zur Förderung der Synthese- bzw. Entgiftungsleistungen der Leber, einem Mittel zur Behandlung von Müdigkeit, Antriebslosigkeit, Libido-, Potenzstörungen, Depressionen, Lern- und Konzentrations schwäche.

Es hat sich überraschenderweise herausgestellt, dass die erfindungsgemäße Zusammensetzung besonders effektiv für diese Anwendungsgebiete ist.

Weiters ist es günstig, wenn die Zusammensetzung in Form von Tabletten, einer Flüssigkeit, Kapseln, Dragees, Sirup, Lotion, Creme bzw. Pulver vorgesehen ist.

Je nach Einsatzgebiet eignet sich die eine oder andere Form besser, da sich beispielsweise als Nahrungsmittelergänzung Tabletten, Kapseln und Dragees oder Sirup eignen. Im Fall, dass die Zusammensetzung eine Wirkung durch die Haut oder auf der Haut ausüben soll, eignet sich eine Lotion bzw. Creme besonders gut. Dabei kann der Fachmann die notwendigen Hilfsstoffe für die jeweilige Form auswählen.

Ein weiterer Aspekt der vorliegenden Erfindung betrifft ein Verfahren zur Herstellung einer erfindungsgemäßen Zusammensetzung, wie oben beschrieben, wobei das Antioxidans A, das Antioxidans B sowie gegebenenfalls eine sauerstoffmaskierende Substanz miteinander vermischt werden und gegebenenfalls die Zusammensetzung zu Tabletten, einer Flüssigkeit, Kapseln, Dragees, Sirup, einer Lotion, Creme bzw. Pulver verarbeitet wird.

Der Schritt des Miteinandervermischens kann beispielsweise in einer Mischtrommel aber auch in jeglichem Behälter automatisch oder händisch durchgeführt werden. Die weitere Verarbeitung erfolgt nach Verfahren wie an sich bekannt. Dabei liegt der Vorteil im erfindungsgemäßen Verfahren darin, dass keine zusätzlichen Vorkehrungen zum Schutz des Antioxidans A notwendig sind, da der Schutz bereits durch das Antioxidans B erfolgt.

Dabei ist es besonders günstig, wenn das Antioxidans A, das Antioxidans B und gegebenenfalls die sauerstoffmaskierende Substanz unter Inertgas vermischt werden. Ein solches Inertgas ist beispielsweise Stickstoff, wodurch der Zutritt vom Sauerstoff zur Zusammensetzung zusätzlich inhibiert oder verringert wird. Damit wird die Gefahr der Oxidation des Antioxidans A weiter herabgesetzt.

Weiters ist es günstig, wenn die Zusammensetzung unter Inertgas verarbeitet wird. Auch hierbei kann das Inertgas beispielsweise Stickstoff sein, wobei es besonders günstig ist, wenn sowohl der Schritt des Vermischens als auch der Schritt des Verarbeitens zu Tabletten, einer Flüssigkeit, Kapseln, Dragees, Sirup, einer Lotion, Creme bzw. Pulver unter Inertgas durchgeführt wird, sodass alle Schritte in einer Weise erfolgen, durch die der Zutritt von Sauerstoff verhindert oder verringert wird.

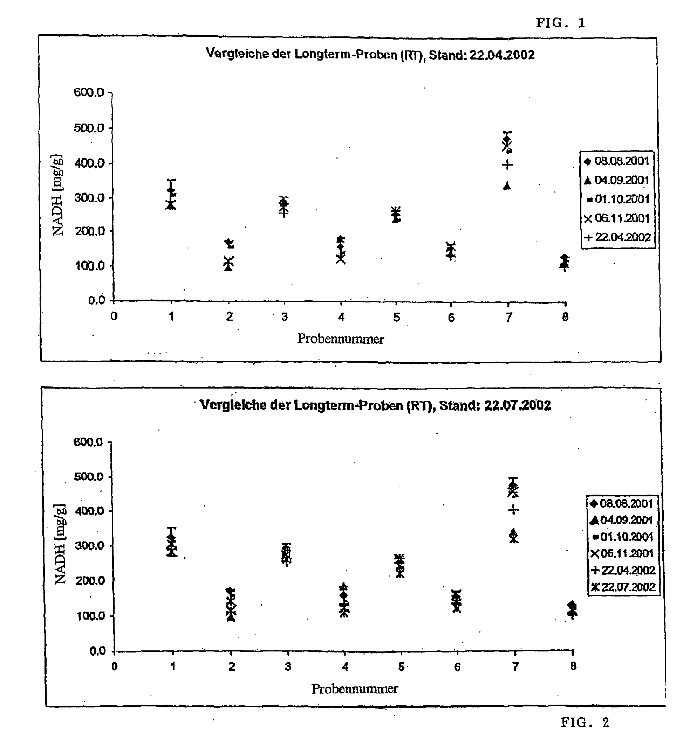

Die vorliegende Erfindung wird nun an Hand der nachfolgenden Beispiele sowie der Figuren näher erläutert, auf die sie jedoch nicht beschränkt sein soll, wobei die Figuren 1 und 2 einen Vergleich der Lagerstabilität verschiedener Zusammensetzungen darstellen.

### Beispiel 1 : Herstellung verschiedener Zusammensetzungen

Im vorliegenden Beispiel wurde NADH (-320mV) als Antioxidans A verwendet. Zur Objektivierung der NADH-protektiven Wirkung durch sauerstoffmaskierende Öle bzw. durch starke Antioxidantien wurden Mischungen von NADH mit einem Tocotrienol-reichen Öl aus gekeimten Weizensamen (in der Folge als Öl bezeichnet) und Chlorophyll als Antioxidans B, das ein Redoxpotential von -660mV aufweist, unter Zuhilfenahme von inertem Aerosil als Hilfsstoff angefertigt:

| | |
|---|---|
| Rezeptur Nr. 1: | NADH 20 mg |
| | Öl 20 mg |
| | Aerosil 20 mg |
| | |
| Rezeptur Nr. 2: | NADH 20 mg |
| | Öl 60 mg |
| | Aerosil 30 mg |
| Rezeptur Nr. 3: | NADH 20 mg |
| | Chlorophyll 20 mg |
| | Aerosil 20 mg |
| | |
| Rezeptur Nr. 4: | NADH 20 mg |
| | Chlorophyll 80 mg |
| | Aerosil 20 mg |
| | |
| Rezeptur Nr. 5: | NADH 20 mg |
| | Öl 20 mg |
| | Chlorophyll 20 mg |
| | Aerosil 20 mg |
| | |
| Rezeptur Nr. 6: | NADH 20 mg |
| | Öl 20 mg |
| | Chlorophyll 60 mg |
| | Aerosil 20 mg |
| | |
| Rezeptur Nr. 7: | NADH 20 mg |
| | Aerosil 20 mg |
| | |
| Rezeptur Nr. 8: | NADH 20 mg |
| | Öl 20 mg |
| | Chlorophyll 60 mg |
| | Aerosil 20 mg |

Die Rezepturen Nr. 1 bis Nr. 7 wurden unter Stickstoff als Inertgas gemischt, anschließend in Hartgelatinekapseln gefüllt und in Polypropylen-Gefäße verpackt.

Rezeptur Nr. 8 wurde ohne Stickstoff als Inertgas gemischt, anschließend - ebenso ohne Stickstoffschutz verkapselt und in Polypropylen-Gefäße gefüllt.

### Beispiel 2 : Prüfung der Lagerstabilität der einzelnen Zusammensetzungen

Alle 8 Prüfmuster wurden unter "Longterm-Bedingungen" bei 25°C und 60 % relativer Feuchtigkeit über einen Zeitraum von 6 Monaten gelagert und in einmonatigen Abständen chemisch/analytisch untersucht. Ziel der Analyse war es, den oxidativen Zerfall von NADH zu NAD⁺ oder anderen Verbindungen festzustellen.

Die analytischen Arbeiten wurden unter folgenden Bedingungen durchgeführt:

### Analysenparameter

| | |
|---|---|
| HPLC: | Agilent 1100 Series mit quaternärer Pumpe, Autosampler, Säulenthermostatisierung und Variable Wavelength-Detektor (VWD) bzw. Fluoreszenzdetektor (FLD) |
| Trennsäule: | LiChrospher® 100 RP-18 (5 µm) 250x4 mm inkl. Guard-Säule 4x4 mm |
| Säulenthermostat: | 25°C |
| Laufmittel: | Mischung aus 900 ml H₂O, 60 ml CH₃CN, 23 ml THF, 1 ml Phosphorsäure und 1,2 g Na-Octansulfonat (NaOSS) |
| Fluss: | 1,5 ml/min |
| Detektion: | VWD: 260 nm |
| | FLD: Excitation: 290 nm, Emission: 395 nm |
| Retentionszeiten: | NADH: 1,04 min |
| | NAD⁺: 1,34 min |

### Standardisierung

Mit Hilfe einer Verdünnungsreihe einer Stammlösung von käuflichem NADH (Pfannenschmidt GmbH) bzw. NAD⁺ (Sigma Aldrich) konnten für beide Substanzen Kalibrationsgeraden für die Bereiche 1 - 50 mg/l (FLD) bzw. 10 - 200 mg/l (VWD) ermittelt werden.

Für die Analyse der Rezepturen wird aufgrund der Konzentration der VWD verwendet, bei geringeren Mengen könnte auch mittels FLD detektiert werden.

### Analysendurchführung

Der Inhalt einer Kapsel wird in eine Flasche mit Schraubverschluss eingewogen, mit 50 ml H₂O dest. versetzt und 1 min kräftig geschüttelt.

Über ein Spritzenfilter (Sartorius Minisart RC 25, 0,45 µm) werden die Schwebstoffe abfiltriert und die Lösung in eine Viale gefüllt, woraufhin mittels HPLC/VWD die Konzentration an NADH und NAD⁺ nach der Methode der externen Standards bestimmt wird.

### Resultate:

Die Auswertungen der NADH-Gehalte der Proben Nr. 1 bis Nr. 8 sind in der Tabelle und in den Figuren gezeigt:

**Tabelle:**

| ***Probennummer*** | ***8.8.2001 NADH*** ***[mg*/*g]*** | ***8.8.2001 Stand. abw.*** | ***04.09.RT NADH*** ***[mg*/*g]*** | ***01.10.RT NADH [mg*/*g]*** | ***6.11.RT NADH [mg*/*g]*** | ***22.04.02RT NADH[mg*/*****g]*** | ***22.7.02RT NADH[mg*/*g]*** |
|---|---|---|---|---|---|---|---|
| 1 | 323.1 | 27.2 | 277.0 | 311.6 | 282.1 | 288.9 | 304.5 |
| 2 | 171.7 | 1.9 | 96.8 | 156.7 | 115.6 | 109.8 | 141.7 |
| 3 | 293.7 | 11.8 | 286.3 | 287.7 | 275.0 | 255.7 | 261.8 |
| 4 | 159.5 | 24.4 | 184.4 | 141.7 | 124.4 | 131.6 | 109.2 |
| 5 | 252.8 | 16.3 | 243.4 | 238.8 | 264.7 | 264.8 | 220.9 |
| 6 | 157.4 | 11.9 | 140.7 | 136.6 | 161.5 | 134.7 | 122.6 |
| 7 | 475.3 | 20.2 | 339.1 | 440.8 | 456.2 | 402.8 | 318.8 |
| 8 | 130.1 | 1.2 | 113.1 | 117.7 | 116.8 | 101.7 | 114.9 |

Wie diese Auswertungen zeigen, ist NADH ohne Zugabe protektiver Substanzen aufgrund seiner Oxidationsempfindlichkeit chemisch instabil (Probe Nr. 7).

Der oxidative Abbau von NADH in Probe Nr. 1 und Probe Nr. 2 zeigt, dass eine Abschirmung von Sauerstoff durch ölige Lösungen (auch wenn diese Tocotrienole enthalten) zwar eine gewisse Schutzwirkung ausübt, einen (wenn auch verzögerten) oxidativen Zerfall von NADH mit zunehmender Zeitdauer jedoch nicht vollständig verhindern kann.

Als optimal erwies sich dagegen die Zugabe von Chlorophyll, das ein äußerst niedriges Redoxpotential von -600mV aufweist und somit offensichtlich in der Lage ist, die Oxidation von NADH (-320mV) durch z.B. Luftsauerstoff zu verhindern (Probe Nr. 3). Als sehr vorteilhaft erwies sich auch die Kombination von Chlorophyll und Tocotrienol-reichem Öl, wie Probe Nr. 5 und Probe Nr. 6 zeigten. Aus den Werten von Probe Nr. 8 ist ersichtlich, dass der antioxidative Schutz von Chlorophyll und Tocotrienol-reichen Ölen offensichtlich so stark ist, dass entsprechende Mischungen sogar ohne Inertgas-Schutz zu Kapseln, Tabletten oder anderen Arzneiformen weiterverarbeitet werden können.

## Patentansprüche

1. Zusammensetzung, **dadurch gekennzeichnet, dass** sie zumindest ein isoliertes, gesundheitsförderndes Antioxidans A mit einem Redoxpotential von unter -180mV, ausgewählt aus der Gruppe bestehend aus NADH, NADPH, FADH₂, FMNH₂, FADH und FMNH, und zumindest ein isoliertes, das Antioxidans A stabilisierende Antioxidans B mit einem Standard-Redoxpotential, das unter dem Standard-Redoxpotential des Antioxidans A liegt, umfasst, wobei das Antioxidans B ein Chlorophyll und/oder ein reduziertes Ferredoxin ist.

2. Zusammensetzung nach Anspruch 1, **dadurch gekennzeichnet, dass** sie weiters eine sauerstoffmaskierende Substanz umfasst.

3. Zusammensetzung nach Anspruch 2, **dadurch gekennzeichnet, dass** die sauerstoffmaskierende Substanz eine ölhältige Substanz ist.

4. Zusammensetzung nach Anspruch 3, **dadurch gekennzeichnet, dass** die sauerstoffmaskierende Substanz eine ölhältige Substanz umfassend zumindest ein weiteres Antioxidans C ist.

5. Zusammensetzung nach Anspruch 4, **dadurch gekennzeichnet, dass** die sauerstoffmaskierende Substanz als Antioxidans C Vitamin E umfasst

6. Zusammensetzung nach Anspruch 5, **dadurch gekennzeichnet, dass** das Vitamin E Antioxidans C Tocotrienol umfasst.

7. Zusammensetzung nach einem der Ansprüche 1 bis 6, **dadurch gekennzeichnet, dass** das Antioxidans A und das Antioxidans B in einem Verhältnis von zwischen 10:1 und 1:10 vorgesehen sind.

8. Zusammensetzung nach einem der Ansprüche 1 bis 7, **dadurch gekennzeichnet, dass** das Antioxidans A und das Antioxidans B in einem Verhältnis von zwischen 3:1 und 1:3 vorgesehen sind.

9. Zusammensetzung nach einem der Ansprüche 1 bis 8, **dadurch gekennzeichnet, dass** sie weiters ein oder mehrere Hilfsmittel umfasst.

10. Verwendung einer Zusammensetzung nach einem der Ansprüche 1 bis 9 zur Nahrungsergänzung.

11. Verwendung einer Zusammensetzung nach einem der Ansprüche 1 bis 9 als Arzneimittel.

12. Verwendung einer Zusammensetzung nach einem der Ansprüche 1 bis 9 zur Herstellung eines Mittels ausgewählt aus der Gruppe bestehend aus einem Mittel zur Förderung der oxidativen Phosphorylierung, einem Mittel zur Förderung der kognitiven, intellektuel len bzw. motorischen Leistungen, einem Mittel zur Förderung der Synthese- bzw. Entgiftungsleistungen der Leber, einem Mittel zur Behandlung von Müdigkeit, Antriebslosigkeit, Libido-, Potenzstörungen, Depressionen, Lern- und Konzentrationsschwäche.

13. Zusammensetzung nach einem der Ansprüche 1 bis 9, **dadurch gekennzeichnet, dass** sie in Form von Tabletten, einer Flüssigkeit, Kapseln, Dragees, Sirup, Lotion, Creme bzw. Pulver vorgesehen ist.

14. Verfahren zur Herstellung einer Zusammensetzung gemäß einem der Ansprüche 1 bis 9, **dadurch gekennzeichnet, dass** das Antioxidans A, das Antioxidans B sowie gegebenenfalls eine sauerstoffmaskierende Substanz miteinander vermischt werden und gegebenenfalls die Zusammensetzung zu Tabletten, einer Flüssigkeit, Kapseln, Dragees, Sirup, einer Lotion, Creme bzw. Pulver verarbeitet wird.

15. Verfahren nach Anspruch 14, **dadurch gekennzeichnet, dass** das Antioxidans A, das Antioxidans B und gegebenenfalls die sauerstoffmaskierende Substanz unter Inertgas vermischt werden.

16. Verfahren nach Anspruch 14 oder 15, **dadurch gekennzeichnet, dass** die Zusammensetzung unter Inertgas verarbeitet wird.

## Claims

1. A composition, **characterized in that** it comprises at least one isolated, health-promoting antioxidant A having a redox potential of below -180mV and selected from the group consisting of NADH, NADPH, FADH₂, FMNH₂, FADH and FMNH, and at least one isolated antioxidant B which stabilizes said antioxidant A and has a standard redox potential ranging below the standard redox potential of said antioxidant A, said antioxidant B being a chlorophyll and/or a reduced ferredoxin.

2. A composition according to claim 1, **characterized in that** it further comprises an oxygen-masking substance.

3. A composition according to claim 2, **characterized in that** said oxygen-masking substance is an oil-containing substance.

4. A composition according to claim 3, **characterized in that** said oxygen-masking substance is an oil-containing substance comprising at least one further antioxidant C.

5. A composition according to claim 4, **characterized in that** said oxygen-masking substance comprises vitamin E as said antioxidant C.

6. A composition according to claim 5, **characterized in that** said vitamin E antioxidant C comprises tocotrienol.

7. A composition according to any one of claims 1 to 6, **characterized in that** said antioxidant A and said antioxidant B are provided at a ratio of between 10:1 and 1:10.

8. A composition according to any one of claims 1 to 7, **characterized in that** said antioxidant A and said antioxidant B are provided at a ratio of between 3:1 and 1:3.

9. A composition according to any one of claims 1 to 8, **characterized in that** it further comprises one or several auxiliary agent(s).

10. Use of a composition according to any one of claims 1 to 9 as a nutritional supplement.

11. Use of a composition according to any one of claims 1 to 9 as a drug.

12. Use of a composition according to any one of claims 1 to 9 for preparing an agent selected from the group consisting of an agent for promoting oxidative phosphorylation, an agent for promoting cognitive, intellectual and motoric abilities, an agent for promoting synthesis and detoxication functions of the liver, an agent for treating fatigue, lethargy, libido and potency disorders, depressions, weaknesses of learning and concentration.

13. A composition according to any one of claims 1 to 9, **characterized in that** it is provided in the form of tablets, a liquid, capsules, coated tablets, a syrup, a lotion, a cream or a powder.

14. A method for preparing a composition according to any one of claims 1 to 9, **characterized in that** said antioxidant A, said antioxidant B as well as, optionally, an oxygen-masking substance are mixed with one another, and the composition is optionally processed into tablets, a liquid, capsules, coated tablets, a syrup, a lotion, a cream or a powder.

15. A method according to claim 14, **characterized in that** said antioxidant A, said antioxidant B and, optionally, said oxygen-masking substance are mixed under inert gas.

16. A method according to claim 14 or 15, **characterized in that** the composition is processed under inert gas.

## Revendications

1. Composition, **caractérisée en ce qu'**elle comprend au moins un antioxydant A isolé bénéfique pour la santé, ayant un potentiel redox en dessous de -180 mV, choisi dans le groupe consistant en NADH, NADPH, FADH₂, FMNH₂, FADH et FMNH, et au moins un antioxydant B isolé, stabilisant l'antioxydant A, ayant un potentiel redox standard qui est inférieur au potentiel redox standard de l'antioxydant A, l'antioxydant B étant une chlorophylle et/ou une ferrédoxine réduite.

2. Composition selon la revendication 1, **caractérisée en ce qu'**elle comprend en outre une substance masquant l'oxygène.

3. Composition selon la revendication 2, **caractérisée en ce que** la substance masquant l'oxygène est une substance huileuse.

4. Composition selon la revendication 3, **caractérisée en ce que** la substance masquant l'oxygène est une substance huileuse qui comprend au moins un autre oxydant C.

5. Composition selon la revendication 4, **caractérisée en ce que** la substance masquant l'oxygène comprend en tant qu'antioxydant C de la vitamine E.

6. Composition selon la revendication 5, **caractérisée en ce que** l'antioxydant C vitamine E comprend du tocotriénol.

7. Composition selon l'une quelconque des revendications 1 à 6, **caractérisée en ce que** l'antioxydant A et l'antioxydant B sont prévus en un rapport compris entre 10:1 et 1:10.

8. Composition selon l'une quelconque des revendications 1 à 7, **caractérisée en ce que** l'antioxydant A et l'antioxydant B sont prévus en un rapport compris entre 3:1 et 1:3.

9. Composition selon l'une quelconque des revendications 1 à 8, **caractérisée en ce qu'**elle comprend en outre un ou plusieurs adjuvants.

10. Utilisation d'une composition selon l'une quelconque des revendications 1 à 9, pour le complément alimentaire.

11. Utilisation d'une composition selon l'une quelconque des revendications 1 à 9, en tant que médicament.

12. Utilisation d'une composition selon l'une quelconque des revendications 1 à 9, pour la fabrication d'un agent choisi dans le groupe consistant en un agent destiné à favoriser la phosphorylation oxydante, un agent destiné à favoriser les capacités cognitives, intellectuelles ou motrices, un agent destiné à favoriser les pouvoirs de synthèse ou de détoxication du foie, un agent destiné au traitement de la fatigue, de l'athymie, de troubles de la libido et de la virilité, de dépressions, de déficiences de l'apprentissage et la concentration.

13. Composition selon l'une quelconque des revendications 1 à 9, **caractérisée en ce qu'**elle est prévue sous forme de comprimés, d'un liquide, de gélules, de dragées, de sirop, lotion, crème ou poudre.

14. Procédé pour la préparation d'une composition selon l'une quelconque des revendications 1 à 9, **caractérisé en ce qu'**on mélange entre eux l'antioxydant A, l'antioxydant B ainsi qu'éventuellement une substance masquant l'oxygène et éventuellement on transforme la composition en comprimés, en un liquide, en gélules, en dragées, en un sirop, en une lotion, crème ou poudre.

15. Procédé selon la revendication 14, **caractérisé en ce que** l'antioxydant A, l'antioxydant B et éventuellement la substance masquant l'oxygène sont mélangés sous un gaz inerte.

16. Procédé selon la revendication 14 ou 15, **caractérisé en ce que** la composition est transformée sous un gaz inerte.
